# EUROPEAN PATENT APPLICATION

(11) **EP 1 219 639 A1**
(43) Date of publication of application: **03.07.2002**
(21) Application number: 00957072.2
(22) Date of filing: 08.09.2000
(51) Int. Cl.: C07K 17/02, C12M 1/00, A61M 1/02, A61M 1/34

(54) **MATERIALS FOR EXTRACORPOREAL CIRCULATION, ADSORBENTS FOR DIABETIC COMPLICATION FACTORS, CONTAINERS FOR ELIMINATING DIABETIC COMPLICATION FACTORS AND METHOD OF ELIMINATING DIABETIC COMPLICATION FACTORS**

(30) Priority: 08.09.1999 JP 25446399
(71) Applicant: TORAY INDUSTRIES, INC., Tokyo 103-8666 (JP)
(72) Inventor: SHIMIZU, Shinji, Moriyama-shi, Shiga 524-0041 (JP); KUBOTA, Masahiro, Kusatsu-shi, Shiga 525-0034 (JP); AKIYAMA, Hideo, Kamakura-shi, Kanagawa 248-0034 (JP); USUI, Mina, Yamato-shi, Kanagawa 242-0006 (JP)
(74) Representative: Coleiro, Raymond
(86) International application number: JP0006172
(87) International publication number: WO0118060

(57) **Abstract**

To provide an available adsorbent that can adsorb and remove diabetic complication factors such as carbonyl stress products, a material for use in extracorporeal circulation includes a water-insoluble carrier and a peptide having part or all of a specific receptor amino acid sequence and being immobilized to the water-insoluble carrier. An adsorbent and a method for removing diabetic complication factors are also provided. Such diabetic complication factors may cause arteriosclerosis and other vascular lesions when the carbonyl stress is pathologically enhanced owing to, for example, deteriorated excretion and metabolic functions in diabetes mellitus or artificial dialysis.

## Description

### Technical Field

The present invention relates to a material for use in extracorporeal circulation. The material comprises a water-insoluble carrier and a peptide having part or all of a specific receptor amino acid sequence, or an antibody specific to a carbonyl stress product as an epitope, in which the peptide or antibody is immobilized to the water-insoluble carrier. The invention also relates to an adsorbent and a unit for the removal of a diabetic complication factor in a fluid to be treated, as well as to a method for the removal of diabetic complication factor using the material, adsorbent or unit.

### Background Art

"Life-style related diseases" have significantly increased and have been perceived as a problem in recent years. Such life-style related diseases include diseases that are caused by, for example, diabetes mellitus or hyperlipidemia, induce arteriosclerosis and other vascular lesions and significantly affect the prognoses of patients in many cases. Specifically, diabetes mellitus causes pathological changes in renal glomeruli with an elapsed diseased period, is attended by renal failure in a more severe stage, and the patient requires dialysis. Diabetic nephropathy overtook chronic glomerulonephritis and climbed to the top of primary diseases of patients undergoing dialysis at the end of 1998. It will certainly contribute to increase the number of patients undergoing dialysis. However, the detailed incidence mechanism of diabetic nephropathy has not yet been revealed and is one of important medical problems even now.

As possible candidates for causes of these vascular lesions, denatured substances such as advanced glycation endproducts (AGEs) have recently received attention. AGEs are supposed to be main causative agents of diabetic complications and are products of advanced glycation. In the advanced glycation, a reduced sugar such as glucose, a metabolite or reaction product thereof in blood is bound to a protein, a lipid, or a low molecular weight substance without catalysis of enzymes. From a chemical view point, this reaction is the reaction between a carbonyl group of the reactant and a nucleophilic reactive group, such as a basic functional group of an amino acid, of the counter reactant. Consequently, the advanced glycation and other denaturation reactions are genetically referred to as "carbonyl stress" (JIN TO TOUSEKI (Kidney and Dialysis), 475:196 (1999)). The denatured reaction products are referred to as carbonyl stress products. It has recently been revealed that oxidative reactions occur in vivo by action of radical bodies such as active oxygen and hydroxy radical and affect the carbonyl stress in a complicated manner to thereby yield the carbonyl stress products (The Japanese Journal of Clinical Dialysis, 14(4):413 (1998)).

For example, Annals of Internal Medicine, 101:527 (1984) and The New England Journal of Medicine, 325:836 (1991) mention the theory that AGEs and other carbonyl stress products are main causative agents of diabetic complications. In addition, the carbonyl stress products also accumulate in patients with excretory functional disorder, such as patients undergoing dialysis.

In those times, the carbonyl stress products as non-enzymatic reaction products between, for example, reduced sugars and proteins were found to exhibit various characteristics. For example, they can emit fluorescence, are brown, can be cross-linked and can undergo dehydration, oxidation, condensation, or rearrangement. However, none of these characteristics can directly account for why these carbonyl stress products cause diseases. Among these carbonyl stress products, AGEs in the blood have conventionally been determined based on their fluorescent characteristics in many cases. For example, Japanese Unexamined Patent Application Publication No. 6-312134 describes the determination of an AGE in human plasma at an excitation wavelength of 390 nm and a fluorescence wavelength of 450 nm according to the method described in pp. 838 of The New England Journal of Medicine, 325:836 (1991). However, the fluorometry technique is significantly affected by non-specific fluorescence of impurities or drugs in blood, although it can be applied to the detection of the advanced glycation in vitro. In addition, it has been revealed that only part of AGEs have the aforementioned fluorescence characteristic. For example, AGEs having different fluorescence characteristics, such as pentosidine with an excitation wavelength of 335 nm and a fluorescence wavelength of 385 nm, have been found (Journal of the American Society of Nephrology, 7(8):1198 (1996)). The simple determination of AGES in human plasma at an excitation wavelength of 390 nm and a fluorescence wavelength of 450 nm is now believed not to reliable. Consequently, when AGEs are determined based on the fluorescence characteristics, a sample is subjected to filtration through a molecular sieve or to chemical treatment, or an AGE structure contributing the fluorescence to be measured is identified. Alternatively, attempts have been made to determine AGEs by specific immunoassays. About ten types of AGEs including carboxymethyl lysine (CML), carboxyethyl lysine (CEL), pentosidine, pyrraline, crossline, fluorolink, imidazolone, X1, and argpyrimidine have been identified. However, there is a theory that all of these AGEs do not always cause diseases as the carbonyl stress products (Kidney International, 51:1170 (1997)). Consequently, the theory that carbonyl stress products such as AGEs cause the vascular lesions becomes further complicated. Thus, no conventional material can selectively and effectively adsorb the carbonyl stress products that induce diabetic complications and other vascular lesions.

There are few treatments that are effective for arteriosclerosis and other vascular lesions such as those in diabetic complications. For patients where nephropathy is complicated, some treatments may retard the progress of the disease but almost no treatment means can effectively and radically cure the disease. In these cases, the prognosis of the patients is further deteriorated. Strong demands are therefore made on effective treatment means for patients with arteriosclerosis and other vascular lesions that are supposed to be caused by AGEs and other carbonyl stress products.

Accordingly, an object of the present invention is to provide a material for use in extracorporeal circulation including a water-insoluble carrier and a peptide having part or all of a specific receptor amino acid sequence, or an antibody specific to a carbonyl stress product as an epitope, immobilized to the water-insoluble carrier, as well as to provide an available adsorbent and method that can adsorb and remove diabetic complication factors such as carbonyl stress products. Such diabetic complication factors may cause arteriosclerosis and other vascular lesions when the carbonyl stress is pathologically enhanced owing to, for example, deteriorated excretion and metabolic functions in diabetes mellitus or artificial dialysis.

### Disclosure of Invention

To achieve the above objects, the present invention provides the following configurations.
"(1) A material for use in extracorporeal circulation, comprising a water-insoluble carrier immobilized a peptide thereto , wherein the peptide has part or all of the receptor amino acid sequence as set forth in SEQ ID NO: 1.
"(2) A material for use in extracorporeal circulation, comprising a water-insoluble carrier immobilized thereto an antibody comprising a carbonyl stress product as an epitope."
"(3) An adsorbent for a diabetic complication factor, which includes a substance immobilized to the water-insoluble carrier, and the substance can specifically remove a diabetic complication factor capable of binding to at least one of the peptide as set forth in (1) or the antibody as set forth in (2)."
"(4) An adsorbent for a diabetic complication factor as set forth in (1), (2) or (3) or a unit comprising the adsorbent housed therein and a method for removing a diabetic complication factor comprising the step of bringing a fluid to be treated into contact with the adsorbent in the unit.

### Best Mode for Carrying Out the Invention

The present invention will be illustrated in further detail below.

Adsorbents for a diabetic complication factor for use in the present invention are not specifically limited as long as they each comprise a water-insoluble carrier and a substance that is capable of specifically binding to a diabetic complication factor and is immobilized to the water-insoluble carrier.

The phrase "a diabetic complication factor causes diseases" as used herein means that the diabetic complication factor comes into contact with a cell and induces one or more phenomena such as induction of cytokines, enhancement of cell migration, up-regulation of DNA synthesis, up-regulation of adhesion molecule expression, and induction of oxidation stress caused by activated NF-κB and thereby induces arteriosclerosis, nephropathy, retinopathy, neuropathy, and other vascular lesions. The diabetic complication factors are exacerbation factors observed not only in patients with diabetes mellitus but also in some patients with decreased excretion and metabolic fimctions as in artificial dialysis. Accordingly, the diabetic complication factor is a generic name for complication factors that are related to vascular lesions and are observed specifically frequently in patients with diabetes mellitus.

The origins of the diabetic complication factors and other carbonyl stress products are not specifically limited, as long as they undergo carbonyl stress reactions as diabetic complication factors. For example, substances exposed to carbonyl stress to thereby yield the carbonyl stress products include lipoproteins and other lipid related proteins and lipids themselves, in addition to proteins. They also include low molecular weight metabolites each having a molecular weight of less than or equal to 10000 such as peptide ingredients and hormones, polysaccharide-derived ingredients such as nucleic acids, and vitamins, as well as amino acids, amino acid-related substances, saccharide-related substances, lipid-related substances, and other metabolites.

Of the diabetic complication factors, reactive substances that induce non-enzymatic denaturation reactions such as carbonyl stress are not specifically limited, as long as they can be formed in vivo. Specifically, such reactive substances include highly reactive compounds such as malondialdehyde, glyoxal, methylglyoxal, and 3-deoxyglucosone, as well as substances derived from glucose, fructose, ribose and other reduced sugars, and phosphate ester metabolites of these reduced sugars. The aforementioned non-enzymatic induction of denaturation is generally referred to as "carbonyl stress" in some cases.

Substances that induce carbonyl stress and other denaturation reactions in vivo include, but are not limited to, the reactive carbonyl compounds, as well as active oxygen, hydroxy radical, nitric oxide and metabolites thereof, and other oxidative radicals and compounds, and other types of compounds. Oxidation reaction has received attention in denaturation related to lipids and has recently been found to be significantly responsible to the advanced glycation that is supposed to cause diabetic complications. Specifically, the carbonyl stress products and other non-enzymatically denatured substances are formed in a manner of very complicated reaction.

All the non-enzymatically denatured substances formed in vivo do not always cause diseases, as described above. According to conventional methods for the detection of the carbonyl stress products and other non-enzymatically denatured substances as the diabetic complication factors, the diabetic complication factors causing diabetic complications cannot significantly be determined directly. It is therefore preferred to selectively remove the non-enzymatically denatured substances having physiological activities as the diabetic complication factors.

Among these diabetic complication factors, non-enzymatically denatured substances bind to specific receptors on cells to thereby cause diseases. Most of such receptors are generally called as scavenger receptors. Some of the receptors naturally having other functions, such as galectin-3 and receptor for AGE (RAGE), interact with the carbonyl stress products and other non-enzymatically denatured substances and thereby cause diseases. Specifically, RAGE induces the onset of the major three complications of diabetes mellitus, i.e., nephropathy, retinopathy and neuropathy, and promotes arteriosclerosis. Animal trials have proven that RAGE interacts with a specific substance that is enhanced under diabetes mellitus condition and induces diabetic complications and other angiopathy (42nd Annual Meeting of Japan Diabetes Society). RAGE is supposed to play the most important role to make the diabetic complication factors cause diseases, but the receptors for use in the present invention are not limited to these substances.

Recent studies have pointed out that the diabetic complication factors that can interact with RAGE also include other substances than the carbonyl stress products and other non-enzymatically denatured substances. Specifically, not only the non-enzymatically denatured substances but also a group of inflammation markers including HMG-1, serum amyloid A, and S100/calguranulin superfamily can interact with RAGE even under conditions in which carbonyl stress is morbidly enhanced as in diabetes mellitus. These substances are generically referred to as extracellular newly identified RAGE-binding protein (EN-RAGE) (Cell, 97:889 (1999)). The substance called as "S100/calguranulin superfamily" is produced by vascular endothelial cells, macrophages derived from monocytes, and lymphocytes. The immune system is enhanced under conditions in which carbonyl stress is morbidly enhanced as in diabetes mellitus. The non-enzymatically denatured substances are supposed to play some roles in this phenomenon.

Some recent studies have reported that patients with arteriosclerosis induced by, for example, diabetes mellitus show an increased antibody titration of a group of AGEs, i.e., CML and other carbonyl stress products in the blood, and that patients undergoing dialysis show an increased antibody titration of lipid peroxides and other denatured lipids in addition to the aforementioned antibody titration. The carbonyl stress products and other non-enzymatically denatured substances may possibly be antigen-presenting, induce chronic inflammation and enhance angiopathy. The substance "S100/calguranulin superfamily" is a possible candidate which plays some role in the root of the aforementioned phenomenon. This substance is produced when a non-enzymatically denatured substance having antigenicity directly or indirectly stimulates vascular endothelial cells, monocyte-derived macrophages, or lymphocytes. Various AGEs having antigenicity have been reported in addition to the above substances and are not specifically limited in the present invention.

Specifically, the diabetic complication factors directly interact with RAGE and other receptors that will cause diseases or indirectly interact with them, for example, by stimulating the immune system due to their antigenicity and thereby enhancing the production of a group of substances identified as "EN-RAGE". To specifically remove these diabetic complication factors, a binding ligand should preferably be a substance at least having a unit of the causative receptor capable of binding to diabetic complication factors, or an antibody that is specific to the epitope unit of the carbonyl stress products and other non-enzymatically denatured substances having antigenicity.

Recent studies have revealed that RAGE enhances not only the onset of diabetic complications but also the formation of amyloids as a result of the interaction with serum amyloid A, amylin, β amyloid, and other substances (Nature Medicine, 6:643 (2000)). HMG-1, a RAGE-binding substance, has been reassessed as a highly lethal late mediator observed in sepsis and as a substance that enhances metastasis and proliferation of cancer cells, in addition to as a substance that enhances neurite outgrowth of neurons, indicating that RAGE is involved in various pathoses. In other words, RAGE-immobilized materials, if provided, are capable of treating diabetic complications, treating Alzheimer's Disease and other amyloid-related diseases, and treating sepsis and cancers. Such diabetic complications cannot be treated with conventional drugs. To provide these materials is therefore very significant.

In order to provide a material for use in extracorporeal circulation which carries an immobilized RAGE or antibody, a ligand immobilized to a water-insoluble carrier should be available at low costs, as it is derived from a protein. A target protein-derived ligand can now be produced by genetic recombination or cell technological techniques. Proteins derived from Mammalia have conventionally been expressed using *Escherichia coli* and other bacteria as hosts but have not always been succeeded. Specifically, membrane proteins, antibodies, and other high molecular weight proteins cannot significantly be produced with the aid of bacteria. When the target membrane protein cannot generally stably be expressed in large amounts due to their insolubility, a required activity can be obtained without the expression of the whole sequence of the membrane protein in some cases. In extreme cases, only a domain capable of binding to the diabetic complication factor may selectively be expressed. In general, the minimum number of amino acids constituting such an active domain is supposed to range from 30 (corresponding to 7% of the whole sequence of human RAGE) to 50 (corresponding to 12% of the whole sequence of human RAGE). RAGE as the adsorption ligand for the diabetic complication factors can be obtained by cloning from a human, bovine, rat and mouse, but such RAGES derived from different species exhibit some interspecies mutation. RAGE is initially identified as a multi-ligand receptor and can be used as the adsorption ligand even when it has a receptor amino acid sequence exhibiting some interspecies mutation, as long as it is capable of binding to the diabetic complication factors. RAGE binds to the diabetic complication factors in an extracellular domain, and a preferred ligand is one derived from humans for its high biocompatibility and low antigenicity. For these reasons, an adsorption ligand having an amino acid sequence corresponding to 74% or more of one derived from human can be used as the ligand even when it exhibits interspecies mutation. When the ligand is derived from *Escherichia coli* and another bacterium as an expression host, the ligand should have a molecular weight of less than or equal to about 20000 for its stable expression in some cases. In these cases, the domain should preferably be narrowed down to a domain required for biding to the diabetic complication factors. Consequently, when a ligand is obtained from a bacterium as a host, it should preferably have an amino acid sequence of equal to or more than 40% of one derived from human, and the resulting ligand is capable of binding to the diabetic complication factors and can relatively stably be expressed. When the adsorption ligand is used in an application, in which the antigen presentation of the ligand becomes a problem as in the whole blood, the ligand is preferably one derived from human. However, RAGE is a receptor exhibiting gene polymorphism and having four possible points of amino acid mutation in the extracellular domain. In consideration of this characteristic of RAGE, the ligand should more preferably have equal to or more than 84% (in consideration of four amino acid residues due to gene polymorphism and the whole extracellular domain sequence of human RAGE) or equal to or more than 85% (in consideration of the whole extracellular domain sequence of human RAGE) of the whole receptor sequence derived from human, provided that the whole of the extracellular domain sequence of human RAGE is used. The resulting expressed ligand has a molecular weight much far exceeding 20000 and its expression may become difficult. Even in this case, the ligand can be expressed by genetic recombination using insect cells.

To easily isolate and purify part or all of the sequence of RAGE as a recombinant, an artificial sequence can be inserted into the gene. Alternatively, the resulting RAGE-derived ligand can be purified on a column including an anti-RAGE antibody. The ligand can easily be purified by inserting an artificial sequence comprising a natural amino acid, such as His-Tag, GST, Fc, protein A, and protein G. When an antibody having an amino acid sequence can be used, an amino acid sequence capable of presenting antigenicity can be used as the artificial sequence. Only one amino acid should preferably be modified to impart novel antigen presentation to the recombinant. This technique can minimize the effects of folding of the host recombinant and can minimize changes in solubility and other physicochemical properties of the recombinant. The effects of folding must be avoided when the artificial sequence comprising a natural amino acid is inserted into the gene. The artificial sequence comprising a natural amino acid is preferably inserted at the C-terminus when the target protein is a membrane protein but may be inserted at the N-terminus or into the domain for efficient expression, for normal folding non-in situ, or depending on the application of the material. The artificial sequences each comprising a natural amino acid are not specifically limited to those mentioned above and also include other artificial sequences comprising a natural amino acid and exhibiting high efficiency. Among these artificial sequences, a suitable artificial sequence comprising a natural amino acid is preferably inserted into the gene, depending on the application thereof. Non-natural amino acids such as derivatives of natural amino acids can also be used as the amino acids to be inserted without problems.

Monoclonal antibodies can be obtained by cell fusion techniques using myeloma cells. Such monoclonal antibodies are more preferred as the ligand than antisera and polyclonal antibodies, since the latter show lot-to-lot variation. To relatively thoroughly detect or determine the non-enzymatically denatured substances, however, the antisera or polyclonal antibodies are preferably used although the precision is somewhat decreased. This is because the non-enzymatically denatured substances among the diabetic complication factors may include unknown structures that are involved in RAGE-binding or gene expression. Monoclonal antibodies against CML, pyrraline, pentosidine, and argpyrimidine are commercially available and can be used as the ligand. However, other antibodies against the other non-enzymatically denatured substances can be used as the ligand, in addition to the aforementioned antibodies. The ligands are not specifically limited and include, for example, ligands comprising bioorganic substances such as proteins or peptides, as well as chemically synthesized ligands for the adsorption of diabetic complication factors.

The ligands for use in the present invention are not specifically limited to the aforementioned antibodies including polyclonal antibodies and monoclonal antibodies obtained by cell fusion technique and to components derived from membrane proteins obtained by genetic recombination technique using insect cells. There are economically promising methods for the expression of membrane proteins and proteins that are difficult to express by conventional methods. The ligands also include the products of these methods.

In the above description, part or all of a receptor or an antibody capable of binding to bioorganic substances such as carbonyl stress products is used as the ligand. In consideration of production costs and stably supply, chemically synthesized ligands are generally advantageously used. However, chemically synthesized ligands having affinity to bioorganic substances capable of binding to the receptor or antibody as described in claim 1 or 5 can only be found after much trial and error.

After intensive investigations, the present inventors have found that the chemically synthesized ligands having affinity to bioorganic substances capable of binding to the receptor or antibody as described in claim 1 or 5 should satisfy at least one of, preferably two or more of, the following requirements:
(1) the ligand includes a cationized atom;
(2) the ligand includes a reactive amine; and
(3) the ligand is represented by the following formula [I]:

   (Water-insoluble Carrier)-(Y)a-Z [I]
wherein Y is a functional group of an amide or a ketone;
Z is represented by: -(cyclic compound 1)l-(open-chain compound)m-(cyclic compound 2)n-NH2 and is a functional group having at least one carbon atom; and
a, l, m, and n are each 0 or an integer of equal to or more than 1.

The present inventors have also found that a quaternary amine as the cationized atom in the requirement (1) has the highest affinity to carbonyl stress products but that the ligand may not always have a cationic structure at around physiological pH. They have found that at least one nitrogen atom having a positive charge is required to form a cationic structure at around physiological pH. However, such positively charged atoms are not specifically limited to nitrogen atoms, as long as they are positively charged, and include carbon atoms, silicon atoms, phosphorus atoms, and organic metals including inorganic compounds. To form a non-cationic structure at around physiological pH, reactive amines and other substances having an electron-withdrawal functional group such as hydroxyl group or amino group in the vicinity of a slightly positively charged functional group, i.e., at the alpha position or beta position, have higher affinity as in the requirement (2). Some nonionic positively charged functional groups form an ionic structure by action of an electron-withdrawal functional group in the vicinity thereof, but these functional groups can be used without problems. Such positively charged functional groups are not specifically limited and include, for example, secondary and tertiary amines, i.e., imines, quaternary amines, i.e., ammonium salts, and other aliphatic nitrogen-containing functional groups, pyrrolidine, pyrazolidine, piperidine, piperazine, and other nitrogen-containing cyclic aliphatic functional groups, as well as pyrrole, pyrroline, pyrazole, imidazole, triazole, pyridine, pyridazine, pyrimidine, pyrazine, triazine, indole, benzimidazole, purine, quinoline, carbazole, acridine, phenanthroline, and other heterocyclic aromatic compounds each having a double bond on its ring structure. The functional groups may also be of a structure such as oxazole, thiazole, morpholine, phenothiazine, and other cyclic aliphatic or aromatic functional groups having oxygen, sulfur, and other highly electronegative atoms as in a toluidine ring. The electron-withdrawal functional groups in the vicinity of the positively charged functional groups are not specifically limited and include, for example, hydroxyl group, carboxyl group, carbonyl group, acetyl group, nitro group, ester groups, halogen groups, sulfonic groups, phosphatic groups, phenyl group, and other aromatic functional groups.

When the ligand is brought into contact with the whole blood, it preferably has a nonionic positively charged structure that is believed to have good biocompatibility. However, the ionicity of the ligand becomes trivial, when a specimen to be treated is not the whole blood or is subjected to pretreatment such as plasma separation, and therefore is not specifically limited herein.

Ligands satisfying the requirement (3) are preferred for the better blood compatibility. Among them, those having an aromatic amine such as 4-aminodiphenylmethane group in the group Z in Chemical Formula [I] are most preferred. Aromatic groups for use herein are not specifically limited and include 6-membered rings, as well as 5- to 10-membered rings. The cyclic compounds 1 and 2 and the open-chain compound in the group Z are not specifically limited to hydrocarbon compounds.

Specifically, the cyclic compounds 1 and 2 in the group Z are not specifically limited and include cyclohexane, cyclopentane, and other alicyclic compounds, as well as derivatives of alicyclic compounds carrying, for example, methyl group, ethyl group or isobutyl group bound thereto. Preferred compounds include aromatic compounds such as phenyl group, diphenylmethyl group, and naphthyl group and also include aromatic derivatives canying halogen groups, alkyl groups, hydroxyl group, nitro group, sulfonic groups, or carboxyl group, of which aromatic amine derivatives are more preferred. These compounds are not specifically limited and include hydrocarbon compounds, as well as pyrrolidine, pyrazolidine, piperidine, piperazine, and other nitrogen-containing cyclic aliphatic functional groups, pyrrole, pyrroline, pyrazole, imidazole, triazole, pyridine, pyridazine, pyrimidine, pyrazine, triazine, indole, benzimidazole, purine, quinoline, carbazole, acridine, phenanthroline, and other heterocyclic aromatic compounds each having a double bond on its ring structure. The compounds may also be of a structure such as oxazole, thiazole, morpholine, phenothiazine, and other cyclic aliphatic or aromatic functional groups having oxygen, sulfur, and other highly electronegative atoms, as in toluidine ring. Likewise, the open-chain compound in the group Z is not specifically limited and includes, for example, ethyl group, hexyl group, octyl group, dodecyl group, and other groups of a straight chain structure; isopropyl group, diethylmethylene group, and other groups of a branched-chain structure; and straight- or branched-chain polyethyleneimine groups, and other groups of nitrogen-containing open-chain structure. Among them, methylene group is typically preferred.

When an adsorbent is designed to remove the diabetic complication factors, the adsorbent more preferably has high affinity to other RAGE-binding substances in addition to the carbonyl stress products. The resulting adsorbent can treat the diabetic complications more effectively and can more advantageously be applied to treatment of Alzheimer's disease, dialysis amyloidosis, juvenile type II diabetes mellitus, and other amyloid-related diseases, immune disorders, sepsis, cancer, and other RAGE-related diseases. The RAGE-binding substances other than the carbonyl stress products include, but are not limited to, HMG-1, S100/superfamily, serum amyloid A, β amyloid, and amylin. Novel RAGE-binding substances will be identified in future, and the present invention can also be applied to any RAGE-binding substances, as long as they are capable of binding to RAGE. The adsorbent preferably remove equal to or more than 30% of such RAGE-binding substances in an adsorption test method described in examples below.

It is more preferred that the adsorbent can remove some other substances together with the RAGE-binding substances from the fluid to be treated. For example, when the diabetic complication of a patient becomes worse, the patient undergoes renal failure and is then forced to undergo artificial dialysis. Dialysis amyloidosis will supervene in some patients with prolonged artificial dialysis, resulting in deteriorated prognosis. In addition, RAGE accelerates the formation of amyloids in vivo. For these reasons, the adsorbent should preferably remove equal to or more than 30% of β microglobulin, a major component of dialysis-induced amyloids, together with the RAGE-binding substances. When the patient undergoes hepatic failure, intracorporeal glucose metabolism balance comes undone and thereby induces diabetic symptoms. In this case, the adsorbent should preferably remove bilirubin together with the RAGE-binding substances, since a level of bilirubin in the blood increases and thereby induces neuropathy. To remove bilirubin, the material preferably has a cationized structure as in the requirement (1). Substances that are preferably removed with the RAGE-binding substances are not specifically limited to these substances, and the ligand can appropriately be selected depending on the type of substances to be removed.

Materials for use in the immobilization of the ligand include, but are not limited to, water-insoluble carriers that can be used in extracorporeal circulation and can undergo graft reactions. They are more preferably materials that can form porous articles each having a large surface area and are further preferably made of a material that can be processed into hollow fibers, since such materials can easily be used with apheresis in, for example, artificial dialysis. Materials that can mitigate stimulation on white blood cells are most preferred. The carriers also include organic-organic or organic-inorganic complex carriers. Such inorganic carriers include glass beads, silica gel, and metallic beads, and such organic carriers include those made of crosslinked poly(vinyl alcohol), crosslinked polyacrylates, crosslinked polyacrylamides, crosslinked polystyrenes, crosslinked polysulfones, and other synthesized polymers; as well as crystalline cellulose, crosslinked agarose, crosslinked dextran, and other polysaccharides.

The shape of the carrier can optionally be selected from, for example, granule-shape, fiber-shape, higher processed articles thereof, and hollow-fiber-shape. The size of the carrier is not specifically limited.

The ligand may not always be immobilized to the surface of the carrier. Specifically, when the ligand can be eluted into the body but exhibits immune tolerance, it can mask internal denatured substances, can mitigate their pathogenicity and can more effectively treat body fluids.

The term "fluids derived from body fluids" as used herein means and includes blood, plasma, sera, ascites, lymph, intra-articular fluids, fractional ingredients derived from these fluids, and other fluid or liquid ingredients derived from living bodies.

The ligand is immobilized to the carrier through a chemical bond or through a physical bond. When the ligand is immobilized through a chemical bond, the surface of the carrier may be modified by a functional group. Such functional groups include, but are not limited to, hydroxyl group, amino group, aldehyde group, carboxyl group, thiol group, silanol group, amide group, epoxy group, halogen groups, succinimide group, and anhydrous acid groups. The ligand may directly be bound to the carrier modified by such a functional group or may be bound to the carrier with the medium of a spacer molecule. When the ligand is immobilized through a physical bond, it is bound to the carrier by action of electropositive binding force between an acidic or basic side chain of the ligand and the carrier or by action of a coordinate bond, for example, through a transition metal. The methods for the immobilization are not specifically limited to the above methods, as long as they can keep the activity of the ligand.

To avoid the detachment of the ligand upon use such as sterilization or treatment, the ligand is preferably immobilized to the carrier through a covalent bond.

The adsorbent of the present invention can be used in treatment according to various techniques. Most simply, blood or another body fluid of a patient is led out from the body, is charged into a blood bag. The adsorbent of the present invention is then mixed with the body fluid in the bag to thereby remove denatured proteins, is removed by filtration through a filter, and the body fluid is then returned to the patient.

Alternatively, the adsorbent is charged into a column and is assembled into an extracorporeal circulation system to thereby adsorb and remove denatured proteins in an online manner. In this case, the whole blood or plasma separated from the blood is allowed to pass through the column. The adsorbent of the present invention can be used in any methods but is most suitably used in the online treatment as mentioned above.

In the extracorporeal circulation system, the adsorbent of the present invention can be used alone or in combination with another apheresis treatment. In the latter case, the adsorbent can be used in combination with dialysis therapy using an artificial dialysis system.

### EXAMPLE 1: Cloning of Human RAGE Extracellular Domain

The upstream and downstream fragments of the extracellular domain were obtained from human lung cDNA library as a template using polymerase chain reaction (PCR) techniques. A total of four PCR primers were designed based on a human RAGE database sequence (GeneBank accession No. M91211), and each two primers were used as upstream and downstream amplification primers, respectively.
- Upstream amplification primers: The numerals in the following parenthesis indicate the positions or base numbers of the primers in RAGE, where A of the initial codon ATG of RAGE is defined as 1:
   S (1-29) and AS (730-749)
- Downstream amplification primers: The numerals in the following parenthesis indicate the positions of the primers in RAGE:
   S (462-481) and AS (1007-1032)
The cleavage sites of restriction enzymes EcoRI and BglII were added to the upstream and downstream primers, respectively. Some of the bases were appropriately replaced to improve the stability of the primers and to further effectively express the gene. In addition, a termination codon (TGA) was added at the downstream end of the extracellular domain to make RAGE soluble.

Two reaction products were subjected to agarose gel electrophoresis. The resulting PCR amplified fragments were recovered from the gel after electrophoresis, were cloned using pUC18 vector, and the base sequences of the PCR products were determined.

### EXAMPLE 2: Gene Insertion into Baculovirus Transfer Vector

Cloning in baculovirus transfer vector was performed in the following manner using pVL 1393 (available from Pharmingen) as a vector. Initially, the vector was cleaved using the restriction enzymes EcoRI and BglII, was subjected to dephosphorylation with an alkaline phosphatase and was then purified. The two, upstream and downstream, fragments of RAGE extracellular domain obtained in Example 1 were cleaved using the following restriction enzymes, and the target fragments were purified. The numerals in the following parentheses are base numbers of the target fragments.
- Upstream: EcoRI/FspI (666 bp)
- Downstream: FspI/BglII (384 bp)
The purified two fragments were inserted downstream the polyhedrin promoter of the pVL 1393 vector. The sizes of the inserted fragments were determined by electrophoresis, and the base sequence of resulting clone was determined to thereby verify that a clone having the target DNA could be constructed.

### EXAMPLE 3: Induction of His-Tag

A fragment carrying a His-Tag added to the downstream end of the extracellular domain was amplified by performing PCR with the use of the RAGE plasmid DNA obtained in Example 2 as a template. A six-time repeated sequence of a codon (CAT) encoding His (histidine) was inserted into the upstream of the termination codon of the downstream amplification primer used in Example 1 and thereby yielded a PCR primer.
- Amplification primer: The numerals in the following parenthesis indicate the positions or base numbers of the primers in RAGE, where A of the initial codon ATG of RAGE was defined as 1:
   S(462-481) and AS(1007-1032 + (CAT)6 + (TGA)2 + BglII site)
Some of the bases were appropriately replaced to improve the stability of the primers and to further effectively express the gene. The resulting gene sequence was found to be a gene sequence comprising a His-Tag fused to the C-terminus of the human RAGE extracellular domain corresponding to the sequence from the N-terminus to the 344 amino acid residue of the receptor amino acid sequence as described in claim 1, where the first amino acid was changed from G to M.

### EXAMPLE 4: Infection of Insect Cells and Expression of Human Extracellular Domain

Sf-9 as an insect cell to be infected with the baculovirus was cultured, was infected with the virus, and a recombinant protein was expressed with reference to the procedures described in BIO INDUSTRY, 10(1):40 (1993).

### EXAMPLE 5: Purification of Human RAGE

A target protein was purified from a culture solution containing the human RAGE extracellular domain obtained in Example 4 using a HisTrap (available from Amersham Pharmacia Biotech Company). Part of the purified human RAGE extracellular domain was bound again to the HisTrap, and the resulting column was disassembled without elution with an elution buffer and thereby yielded a human RAGE-bound carrier (Adsorbent 1). The elution volume of the human RAGE-bound carrier was determined using BCA Protein Assay kit (available from Pierce Chemical Company) to find that the carrier carried the human RAGE at an amount of about 2.26 mg/g-gel.

### EXAMPLE 6: Preparation Method of AGE

A standard sample was prepared with reference to the procedure described in Journal of Biological Chemistry, 15, 267(8):5133 (1992). Bovine serum albumin (BSA) was incubated with glucose at 37°C for six months in the presence of bovine-derived ribonuclease A (available from SIGMA) as an antigen. During the reaction, the pH of the reaction solution was adjusted to between 7 to 7.4 with NaOH. After the completion of reaction, the reaction mixture was dialyzed with PBS and thereby yielded the standard sample.

### EXAMPLE 7: Preparation of Anti-AGE Antibodies

A New Zealand White rabbit (NZW) was immunized with the human RAGE extracellular domain expressed in baculovirus with reference to the procedure in Example 6 and according to the procedure described in Saibokogaku (Cell Technology), Suppl. Anti-peptide Antibody Experimental Protocol, pp. 48. The rabbit was bled from the cervical artery after the completion of immunization to yield an antiserum. The antiserum was purified through a HiTrap Protein G column (available from Amersham Pharmacia Biotech Company). The resulting antibody scarcely reacted with BSA but reacted with the AGE-BSA, indicating that the antibody recognizes an AGE having antigenicity.
The amounts of AGEs in clinical specimens (plasma) were determined by a competitive method using the above-prepared antiserum. A solid-phased antigen was prepared by incubating 100 µl of the AGE-BSA obtained in Example 6 with a solid phase at a concentration of 10 µg/ml for two hours. A standard sample for standard curve was prepared in a similar manner as above using the AGE-BSA obtained in Example 6 at a concentration of 1 U/ml. To determine the RAGE amounts, 25 µl of a buffer, 25 µl of a sample to be measured, and 50 µl of the diluted antibodies were allowed to react one another, an antibody recognizing the solid antigen was allowed to react with a peroxidase-labeled anti-rabbit immunoglobulin G (IgG) antibody (available from Kappel) and was developed to thereby determine the amount of AGE. The results are shown below.
- Healthy persons (n=6): 3.92 ± 1.27 U/ml
- Patients with diabetic nephropathy (n = 10: blood sampled before dialysis): 5.72 ± 0.82 U/ml
The results showed a statistically significant difference in t-test, indicating that the obtained antibody recognizes the AGE antigen contained in a higher concentration in the plasma of the patients than in the healthy persons.

### EXAMPLE 8: Immobilization of Antibody and Human RAGE

An adsorbent (Adsorbent 2) was prepared in the following manner. To 1 ml of Affi-Gel 10 (available from Bio-Rad Laboratories), an agarose gel carrying an N-hydroxysuccinimide ester group induced via a spacer of a length corresponding to ten atoms, a solution of 1.42 mg of the polyclonal antibody obtained in Example 7 in 1 ml of 0.1 M HEPES-NaOH buffer (pH 7.5) was added, and the resulting mixture was slowly stirred at 4°C over night. The mixture was then allowed to react with 0.1 ml of 1 M ethanolamine-HCl (pH 8.0) at room temperature for 1.5 hours to thereby inactivate unreacted N-hydroxysuccinimide ester group. The mixture was then rinsed alternatively with three portions of 1 ml of 0.1 M acetic acid-NaOH (pH 4.0) and 1 ml of 0.1 M carbonic acid-NaOH (pH 9.0) each containing 0.5 M NaCl, and was ultimately equilibrated with a phosphate buffer. The resulting adsorbent (Adsorbent 2) carried the antibody immobilized at an concentration of about 1.31 mg/g-gel as calculated backward from the amount of the remained antibody.

Separately, another adsorbent (Adsorbent 3) was prepared in the following manner. Human RAGE was immobilized to Affi-Gel 10 in a similar manner as in the immobilization of the antibody. In this procedure, 1.71 mg of the purified human RAGE obtained in Example 5 was used per 1 ml of the Affi-Gel 10 carrying an N-hydroxysuccinimide ester group via a spacer of a length corresponding to ten atoms. The human RAGE was immobilized at a concentration of about 1.42 mg/g-gel as calculated backward from the amount of the human RAGE remained in the solution.

### EXAMPLE 9: Preparation of Amidomethylated Fiber

An islands-in-a-sea type composite fiber (thickness: 2.6 deniers, number of islands: 16) described in U.S. Patent No. 4661260 comprising 50 parts by weight of a sea ingredient (a mixture of 46 parts by weight of a polystyrene and 4 parts by weight of a polypropylene) and 50 parts by weight of an island-ingredient (a polypropylene) was allowed to react with a mixture containing 50 grams of N-hydroxymethyl-α-chloroacetamide, 400 grams of nitrobenzene, 400 g of 98% sulfuric acid, and 0.85 grams of paraformaldehyde at 20°C for one hour. The resulting fiber was rinsed with nitrobenzene, the reaction was terminated with water, the resulting mixture was rinsed again with methanol and thereby yielded an α-chloroacetamidomethylated crosslinked polystyrene fiber (hereinafter briefly referred to as "AMPSt fiber").

### EXAMPLE 10: Immobilization of Antibody and Human RAGE to Amidomethylated Fiber

The AMPSt fiber obtained in Example 9 was further rinsed with water and thereby yielded 0.5 g of an AMPSt fiber in terms of dry weight. The human RAGE obtained in Example 5 and the anti-AGE antibody obtained in Example 7 were replaced with 0.1 M sodium bicarbonate aqueous solution in a desalting column and thereby yielded an antibody solution or a human RAGE solution, respectively. A total of 0.5 gram of the AMPSt fiber was allowed to react with 1.08 mg/8-ml of the above-prepared human RAGE solution or with 1.28 mg/5-ml of the antibody solution, respectively, at 37°C for two hours with gentle shaking. The amounts of the immobilized antibody and human RAGE were determined based on absorbencies before and after reaction. The antibody-immobilized fiber carried 0.82 mg/g-fiber of the antibody (Adsorbent 4). The human-RAGE-immobilized fiber carried 1.18 mg/g-fiber of the human RAGE (Adsorbent 5). Each of the immobilized fibers was replaced with 5 ml of 0.1 M ethanolamine-HCl (pH 8.0), was allowed to react at room temperature for 1.5 hours, was then rinsed alternatively with three portions of 1 ml of 0.1 M acetic acid-NaOH (pH 4.0) and 1 ml of 0.1 M carbonic acid-NaOH (pH 9.0) each containing 0.5 M NaCl, and was ultimately equilibrated with a phosphate buffer. The resulting fibers were subjected to an adsorption test below.

### EXAMPLE 11: Preparation of Non-bioorganic Molecule-immobilized Beads

A total of 12 ml (sedimentation volume, corresponding to a dry weight of 1.0 gram) of chitosan beads (available from Fuji Spinning Co., Ltd. under the trade name of Chitopearl AL-01) having a diameter of particle of 0.1 mm were stirred in dimethylformamide for 20 minutes. This procedure was repeated four times to thereby completely replace contained water with dimethylformamide.

The resulting beads were gradually added to a solution of 10 g of hexamethylene diisocyanate in 1 liter of dimethylformamide, were allowed to react at room temperature for one hour with stirring, were then taken out from the solution, were placed in 1 liter of fresh dimethylformamide and were rinsed for 20 minutes. The rinsing procedure was repeated three times to thereby completely remove unreacted hexamethylene diisocyanate. The beads were then rinsed with water four times to thereby replace dimethylformamide with water, were allowed to react with 0.1 M sodium hydroxide aqueous solution at room temperature for 20 minutes with stirring to thereby hydrolyze an isocyanate group into a primary amino group, were further rinsed with water four times, were immersed in water at 80°C for 20 minutes and thereby yielded chitosan beads (Adsorbent 6) having the following constitutional formula:

(Chitosan bead)-CONH-(CH2)6-NH2 [II]

Separately, the chitosan beads AL-01 (dry weight: 1.0 gram) used above were subjected to 20-minute stirring operation in dimethylformamide four times to thereby replace contained water with dimethylformamide. The beads were then gradually added to a solution of 15 g of 4,4'-diphenylmethane diisocyanate in 1 liter of dimethylformamide, were allowed to react at room temperature for one hour with stirring, were then taken out from the solution, were rinsed with 1 liter of fresh dimethylformamide for 20 minutes. The rinsing procedure was repeated three times to thereby completely remove unreacted 4,4'-diphenylmethane diisocyanate. The beads were then rinsed with water four times to thereby replace dimethylformamide with water, were allowed to react with 0.1 M sodium hydroxide aqueous solution at room temperature for 20 minutes with stirring to thereby hydrolyze an isocyanate group into a primary amino group, were rinsed with water four times, were immersed in water at 80°C for 20 minutes and thereby yielded chitosan beads (Adsorbent 7) having the following constitutional formula:

(Chitosan bead)-CONH-C6H4-CH2-C6H4-NH2 [III]

Likewise, cellulose beads (Cellulofine GCL-200cc; dry weight: 1.0 g) were subjected to 20-minute stirring operation in dimethylformamide four times to thereby completely replace contained water with dimethylformamide.

The beads were then gradually added to a solution of 15 g of 4,4'-diphenylmethane diisocyanate in 1 liter of dimethylformamide, were allowed to react at room temperature for one hour with stirring, were taken out from the solution, were rinsed with 1 liter of fresh dimethylformamide for 20 minutes. The rinsing procedure was repeated three times to thereby completely remove unreacted 4,4'-diphenylmethane diisocyanate. The beads were then rinsed with water four times to thereby replace dimethylformamide with water, were allowed to react with 0.1 M sodium hydroxide aqueous solution at room temperature for 20 minutes with stirring to thereby hydrolyze an isocyanate group into a primary amino group, were then further rinsed with water four times, were immersed in water at 80°C for 20 minutes and thereby yielded cellulose beads (Adsorbent 8) having the following constitutional formula:

(Cellulose bead)-CONH-C6H4-CH2-C6H4-NH2 [IV]

Separately, the chitosan beads AL-01 (dry weight: 1.0 gram) used above were subjected to 20-minute stirring operation in dimethylformamide four times to thereby completely replace contained water with dimethylformamide. The beads were then gradually added to a solution of 15 g of biphenyl-4,4'-dicarbonyl chloride in 1 liter of dimethylformamide, were allowed to react at room temperature for one hour with stirring, were then taken out from the solution, were rinsed with 1 liter of fresh dimethylformamide for 20 minutes. The rinsing procedure was repeated three times to thereby completely remove unreacted biphenyl-4,4'-dicarbonyl chloride. The beads were rinsed with water four times to thereby replace dimethylformamide with water, were allowed to react with 0.1 M sodium hydroxide aqueous solution at room temperature for 20 minutes with stirring to thereby hydrolyze an isocyanate group into a primary amino group, were further rinsed with water four times, were immersed in water at 80°C for 20 minutes and thereby yielded chitosan beads (Adsorbent 9) having the following constitutional formula:

(Chitosan bead)-CO-C6H4-C6H4-NH2 [V]

### EXAMPLE 12: AGE Adsorption Test on Adsorbents

The adsorbents obtained in Examples 5, 8, 10 and 11, and commercially available adsorbents were subjected to AGE-adsorption test. As a sample, 0.5% BSA-phosphate buffer containing 10 µg/ml of the AGE-BSA obtained in Example 6 was used. When an adsorbent under test was a gel, 900 µl of the sample was added to 100 µl in terms of sedimentation volume of the adsorbent. When an adsorbent under test was a fiber, 1 ml of the sample was added to 50 µg in terms of a dry weight of the AMPSt fiber. The resulting mixture was slowly shaken in an incubator at 37°C for two hours. The obtained reaction mixture was separated by centrifugation at 3000 rpm for five minutes to sediment the adsorbent, and the amount of AGE in the supernatant was determined according to the immunoassay technique described in Example 7.

As the commercially available adsorbents, TSK-gel QAE-TOYOPEARL 650M (available from Tosoh Corp.: Adsorbent 10) as a cationic carrier and Amino-Cellulofine (available from CHISSO CORPORATION: Adsorbent 11) as a reactive-amine-immobilized carrier were used. Cationic carriers having affinity to AGE are not specifically limited to those mentioned above and include any cationic carriers, as long as they are cationic under physiological pH conditions. Likewise, reactive-amine-immobilized carriers are not specifically limited, as long as they exhibit similar reactivity under physiological conditions.

| AGE Adsorbent | AGE Adsorption Rate (%) |
|---|---|
| • RAGE-immobilized beads (Adsorbent 1) | 54.2 |
| • Antibody-immobilized beads (Adsorbent 2) | 68.3 |
| • RAGE-immobilized beads (Adsorbent 3) | 52.1 |
| • Antibody-immobilized fiber (Adsorbent 4) | 65.3 |
| • RAGE-immobilized fiber (Adsorbent 5) | 51.8 |
| • Modified chitosan beads (Adsorbent 6) | 45.5 |
| • Modified chitosan beads (Adsorbent 7) | 65.7 |
| • Modified cellulose beads (Adsorbent 8) | 51.7 |
| • Modified chitosan beads (Adsorbent 9) | 50.9 |
| • Cationic beads (Adsorbent 10) | 90.6 |
| • Reactive-amine-immobilized beads (Adsorbent 11) | 70.5 |

These adsorbents exhibited good selectivity to AGE. Specifically, the RAGE- or antibody-immobilized carriers, as well as the modified beads, exhibited good affinity to AGE. Especially, the cationic beads and reactive-amine-immobilized beads exhibited satisfactory affinity to AGE.

### COMPARATIVE EXAMPLE 1: AGE Binding Test 2 on Adsorbents

The material Affi-Gel 10 used in Example 8 was allowed to react with 0.1 ml of 1 M ethanolamine-HCl (pH 8.0) at room temperature for 1.5 hours and thereby yielded a carrier containing an inactivated N-hydroxysuccinimide ester group (Adsorbent 12). The above-prepared adsorbent and the AMPSt fiber (Adsorbent 13) prepared in Example 9 were subjected to AGE-adsorption test in a similar manner as in Example 11. In addition, TSK-gel AF-Blue TOYOPEARL 650M (available from Tosoh Corp.: Adsorbent 14) was also subjected to AGE-adsorption test to verify that the measured adsorption rates did not depend on the affinity to BSA serving as a carrier for AGE.

| Adsorbent | AGE Adsorption Rate (%) |
|---|---|
| • Inactivated beads (Adsorbent 12): | 3.2 |
| • AMPSt fiber (Example 13): | 5.3 |
| • BSA-philic carrier (Example 14) | 20.5 |

These results show that the tested adsorbents did not exhibit significant affinity to AGE, indicating that specific affinity to AGE is obtained by immobilizing a ligand having specific affinity to the diabetic complication factors.

### COMPARATIVE EXAMPLE 2: AGE Adsorption Test 3 on Adsorbents

The adsorbents obtained in Examples 5, 8, 10 and 11 and commercially available adsorbents were subjected to AGE adsorption test. In this test, the AGE-BSA obtained in Example 6 was further purified using an RAGE-binding affinity column to pure RAGE-binding AGE and was then subjected to test as a standard sample. Specifically, 3 ml of Adsorbent 3 obtained in Example 8 as the RAGE-binding affinity gel was charged into an open column and was rinsed with 10 ml of phosphate buffered saline (PBS). The AGE-BSA obtained in Example 6 was added to the column at a concentration of 30 mg/ml, and non-RAGE-binding AGE-BSA was then removed with 50 ml of PBS. The AGE-BSA bound to column was recovered by adding 0.1 M glycine-HCl buffer (pH 2.0), was neutralized with 1 M Tris-HCl buffer (pH 9.0), was sufficiently dialyzed with PBS and thereby yielded the target RAGE-binding AGE. The concentration of the RAGE-binding AGE was about 4.71 mg/ml, as determined by using a BCA protein assay kit (available from Pierce Chemical Company).

The adsorption test was performed in a similar manner as in Example 12. Specifically, 0.5% BSA-phosphate buffer containing 10 µg/ml of the above-prepared RAGE-binding AGE was used as a sample. When an adsorbent under test was a gel, 900 µl of the sample was added to 100 µl in terms of sedimentation volume of the adsorbent. When an adsorbent under test was a fiber, 1 ml of the sample was added to 50 µg in terms of a dry weight of the AMPSt fiber. The resulting mixture was slowly shaken in an incubator at 37°C for two hours. The obtained reaction mixture was separated by centrifugation at 3000 rpm for five minutes to sediment the adsorbent, and the amount of AGE in the supernatant was determined according to the immunoassay technique described in Example 7.

As the commercially available adsorbents, TSK-gel QAE-TOYOPEARL 650M (available from Tosoh Corp.: Adsorbent 10) as a cationic carrier and Amino-Cellulofine (available from CHISSO CORPORATION: Adsorbent 11) as a reactive-amine-immobilized carrier were used. Cationic carriers having affinity to AGE are not specifically limited to those mentioned above and include any cationic carriers, as long as they are cationic under physiological pH conditions. Likewise, reactive-amine-immobilized carriers are not specifically limited, as long as they exhibit similar reactivity under physiological conditions.

| AGE Adsorbent | AGE Adsorption Rate (%) |
|---|---|
| • RAGE-immobilized beads (Adsorbent 1) | 71.7 |
| • Antibody-immobilized beads (Adsorbent 2) | 78.3 |
| • RAGE-immobilized beads (Adsorbent 3) | 74.7 |
| • Antibody-immobilized fiber (Adsorbent 4) | 67.9 |
| • RAGE-immobilized fiber (Adsorbent 5) | 61.0 |
| • Modified chitosan beads (Adsorbent 6) | 47.4 |
| • Modified chitosan beads (Adsorbent 7) | 65.9 |
| • Modified cellulose beads (Adsorbent 8) | 53.3 |
| • Modified chitosan beads (Adsorbent 9) | 51.8 |
| • Cationic beads (Adsorbent 10) | 99.2 |
| • Reactive-amine-immobilized beads (Adsorbent 11) | 71.2 |

These adsorbents also exhibited good selectivity to RAGE-binding AGE. Specifically, the RAGE- or antibody-immobilized carriers, as well as the modified beads, exhibited good affinity to the RAGE-binding AGE. The results show that Adsorbents 1 through 11 had better affinity to the RAGE-binding AGE.

### COMPARATIVE EXAMPLE 3: AGE Binding Test 4 on Adsorbents

Adsorbents 12 through 14 obtained in Comparative Example 1 were subjected to adsorption test to the RAGE-binding AGE in a similar manner as in Comparative Example 2. In addition, TSK-gel AF-Blue TOYOPEARL 650M (available from Tosoh Corp.: Adsorbent 14) was also subjected to RAGE-binding AGE adsorption test to verify that the adsorption rates determined according to the adsorption test in Comparative Example 2 did not depend on the affinity to BSA serving as a carrier for AGE.

| Adsorbent | AGE Adsorption Rate (%) |
|---|---|
| • Inactivated beads (Adsorbent 12): | 3.1 |
| • AMPSt fiber (Example 13): | 4.9 |
| • BSA-philic carrier (Example 14) | 18.9 |

These results show that the tested adsorbents did not exhibit significant affinity to RAGE-binding AGE as in Comparative Example 1, indicating that Adsorbents 1 through 11 have specific affinity to the RAGE-binding AGE by immobilizing a ligand having specific affinity to the diabetic complication factors to a carrier.

### EXAMPLE 13: Human Serum Ingredients Adsorption Test on Adsorbents

Adsorbent 7 prepared in Example 11 was subjected to adsorption test of human serum ingredients. Specifically, 1 gram of Adsorbent 7 was added to 10 ml of a fresh human serum and was shaken at 37°C for two hours. The human blood ingredients in the supernatants before and after shaking were measured, and the results are shown below. In this procedure, β2 microglobulin and serum amyloid A were determined by enzyme linked immunosorbent assay (ELISA) and a latex agglutination immunological method, respectively.

| Adsorbate | Adsorption Rate (%) |
|---|---|
| • β2 microglobulin | 81.3 |
| • Serum amyloid A | 42.3 |

The results show that Adsorbent 7 has affinity to β2 microglobulin and serum amyloid A in addition to AGE.

Next, Adsorbent 10 prepared in Example 12 was subjected to adsorption test of human serum ingredients. Specifically, 1 gram of Adsorbent 10 was added to 10 ml of a fresh human serum and was shaken at 37°C for two hours. In this procedure, the adsorption rate of bilirubin was determined by measuring the amounts of bilirubin by an alkaline azobilirubin method before and after shaking.

| Adsorbate | Adsorption Rate (%) |
|---|---|
| • Bilirubin | 70.2 |

The result shows that Adsorbent 10 has affinity to bilirubin in addition to AGE.

### EXAMPLE 14: HMG-1 Adsorption Test on Adsorbent

Adsorbent 7 prepared in Example 11 and Adsorbent 10 prepared in Example 12 were subjected to HMG-1 adsorption test. Specifically, human HMG-1 in 1 ml of 0.5% BSA-phosphate buffer (pH 7.2) was added to a concentration of 100 ng/ml to 100 µl of an adsorbent under test and was shaken at 37°C for two hours. The amounts of HMG- 1 in the supernatants before and after reaction were determined by ELISA.

| Adsorbent | Adsorption Rate (%) |
|---|---|
| • Adsorbent 7 | 45.8 |
| • Adsorbent 10 | 67.9 |

The results show that Adsorbents 7 and 10 have affinity to HMG-1 in addition to AGE.

### EXAMPLE 15: AGE Adsorption Test on Adsorbent-filled Unit

Adsorbent 7 prepared in Example 11 was charged into a cylindrical column having a capacity of 1 ml and having one inlet and one outlet. Filters were mounted to the inlet and outlet in order to avoid washout of the adsorbent. AGE in 10 ml of 0.5% BSA-phosphate buffer (pH 7.2) was allowed to pass through the column filled with Adsorbent 7 at a final concentration of 10 µg/ml at a flow rate of 0.5 ml/min. using a Peristaltic Pump. The AGE removal rates were determined by measuring the AGE concentrations at intervals of 30 minutes by ELISA.

| Time (minute) | Adsorption Rate (%) |
|---|---|
| 0 | 0 |
| 30 | 23.4 |
| 60 | 43.9 |
| 90 | 51.1 |
| 120 | 62.6 |

The results show that the column filled with Adsorbent 7 is capable of removing AGE. The adsorbent charged in the column showed no deformation due to pressure loss during adsorption operation.

### Industrial Applicability

The adsorbents of the present invention each comprise a water-insoluble carrier and a substance capable of specifically binding to diabetic complication factors and being immobilized to the water-insoluble carrier and are adsorbents for diabetic complication factors. In the adsorbents, the substance capable of specifically binding to diabetic complication factors is bound to the water-insoluble carrier through a chemical bond such as a covalent bond or noncovalent bond or though a physical bond. The base materials for the carriers used herein have been used in practical extracellular circulation therapy. The methods of the present invention for removing diabetic complication factors are performed by bringing the adsorbent for diabetic complication factors into contact with a fluid to be treated and are very useful for treatment of angiopathy due to nonenzymatic reactions as in diabetic complications.

## Claims

1. A material for use in extracorporeal circulation, comprising a water-insoluble carrier immobilized a peptide thereto , wherein the peptide has part or all of the receptor amino acid sequence as set forth in SEQ ID NO: 1.

2. The material for use in extracorporeal circulation according to claim 1, wherein the peptide is further inserted with at least one artificial sequence comprising a natural amino acid at an optional position.

3. The material for use in extracorporeal circulation according to claim 1 or 2, wherein the artificial sequence comprising a natural amino acid includes at least one amino acid.

4. The material for use in extracorporeal circulation according to any one of claims 1 to 3, wherein the artificial sequence comprising a natural amino acid is a His-Tag.

5. A material for use in extracorporeal circulation, comprising a water-insoluble carrier immobilized thereto an antibody comprising a carbonyl stress product as an epitope.

6. The material for use in extracorporeal circulation according to claim 5, wherein the epitope has such a structure that can be formed both in vivo and in vitro.

7. The material for use in extracorporeal circulation according to claim 5 or 6, wherein the epitope is generally detected in body fluids of patients with diabetes mellitus in a higher amount than in those of healthy persons.

8. An adsorbent for a diabetic complication factor, comprising a water-insoluble carrier immobilized a ligand thereto, the ligand being capable of binding to at least one of a substance capable of binding to the peptide as claimed in any one of claims 1 to 4 and a substance capable of binding to the antibody as claimed in any one of claims 5 to 7.

9. The adsorbent for a diabetic complication factor according to claim 8, which is the material for use in extracorporeal circulation as claimed in any one of claims 1 to 7.

10. The adsorbent for a diabetic complication factor according to claim 8, wherein the ligand immobilized to the water-insoluble carrier is a non-bioorganic molecule and comprises a cationized atom.

11. The adsorbent for a diabetic complication factor, comprising a water-insoluble carrier immobilized a ligand thereto, wherein the cationized atom according to claim 10 is a nitrogen.

12. The adsorbent for a diabetic complication factor, comprising a water-insoluble carrier immobilized a ligand thereto, wherein the a functional group containing the cationized nitrogen according to claim 10 or 11 is derivable from at least one selected from the group consisting of acyclic or cyclic aliphatic compounds, aromatic compounds, and heterocyclic compounds.

13. The adsorbent for a diabetic complication factor, comprising a water-insoluble carrier immobilized a ligand thereto, wherein the ligand immobilized to the water-insoluble carrier according to claim 8 is a non-bioorganic molecule and comprises a reactive amine.

14. The adsorbent for a diabetic complication factor, wherein the ligand immobilized to the water-insoluble carrier according to claim 8 is represented by the following formula [I].
(Water-insoluble Carrier)-(Y)a-Z [I]
Y: a functional group of an amide or a ketone
Z: -(cyclic compound 1)l-(open-chain compound)m-(cyclic compound 2)n-NH2 and is a functional group having at least one carbon atom
a, l, m, and n: 0 or an integer of equal to or more than 1

15. The adsorbent for a diabetic complication factor according to claim 14, wherein the functional group Y in the compound [I] is bound to at least one of an amino group or a hydroxyl group of the water-insoluble carrier.

16. The adsorbent for a diabetic complication factor according to claim 14 or 15, wherein the open-chain compound in the compound [I] is a hydrocarbon compound.

17. The adsorbent for a diabetic complication factor according to any one of claims 14 to 16, wherein the cyclic compound 2 in the compound [I] is one of an aromatic compound or a heterocyclic compound.

18. The adsorbent for a diabetic complication factor according to any one of claims 14 to 17, wherein the cyclic compound 1 in the compound [I] is one of an aromatic compound and a heterocyclic compound.

19. The adsorbent for a diabetic complication factor according to any one of claims 1 to 18, wherein the immobilization to the water-insoluble carrier is made through a covalent bond, a chemical bond including noncovalent bond, or through a physical bond.

20. The adsorbent for a diabetic complication factor, wherein the water-insoluble carrier according to any one of claims 1 to 19 comprises a polysaccharide or a vinyl aromatic compound.

21. The adsorbent for a diabetic complication factor, wherein the material or the adsorbent, according to any one of claims 1 to 20, can remove at least 40% of a carbonyl stress product.

22. The adsorbent for a diabetic complication factor, wherein the material or the adsorbent, according to claim 21, can remove at least 30% of the substances capable of binding to the peptide as set forth in any one of claims 1 to 4 other than the carbonyl stress product.

23. The adsorbent for a diabetic complication factor, wherein the material or the adsorbent, according to claim 20 or 21, can remove at least 30% of β2 microglobulin.

24. A removal unit for a diabetic complication factor, in which the material or the adsorbent as claimed in any one of claims 1 to 23 is housed.

25. A method for removing a diabetic complication factor, wherein a fluid to be treated is brought into contact with the unit housed with the material or the adsorbent as claimed in any one of claims 1 to 24.

26. The unit and the method for the removal of a diabetic complication factor, wherein the fluid to be treated according to the claim 24 and 25 is a fluid derived from a body fluid.


SEQUENCE LISTING

<110> Toray Industries, Inc.

<120> MATERIAL FOR USE IN EXTRACORPOREAL CIRCULATION, ADSORBENT, REMOVA
L UNIT AND REMOVING METHOD FOR DIABETIC COMPLICATION FACTORS

<130> TD-00062
<150> JP P1999-254463
<151> 1999-9-8
<160> 1

<210> 1
<211> 405
<212> PRT
<213> Homo sapiens
<221> peptide
<400>
Met Ala Ala Gly Thr Ala Val Gly Ala Trp Val Leu Val Leu Ser Leu
1               5                   10                  15

Trp Gly Ala Val Val Gly Ala Gln Asn Ile Thr Ala Arg Ile Gly Glu
            20                  25                  30

Pro Leu Val Leu Lys Cys Lys Gly Ala Pro Lys Lys Pro Pro Gln Arg
        35                  40                  45

Leu Glu Trp Lys Leu Asn Thr Gly Arg Thr Glu Ala Trp Lys Val Leu
    50                  55                  60

Ser Pro Gln Gly Gly Gly Pro Trp Asp Ser Val Ala Arg Val Leu Pro
65                  70                  75                  80
Asn Gly Ser Leu Phe Leu Pro Ala Val Gly Ile Gln Asp Glu Gly Ile
                85                  90                  95
Phe Arg Cys Gln Ala Met Asn Arg Asn Gly Lys Glu Thr Lys Ser Asn
            100                 105                 110
Tyr Arg Val Arg Val Tyr Gln Ile Pro Gly Lys Pro Glu Ile Val Asp
        115                 120                 125
Ser Ala Ser Glu Leu Thr Ala Gly Val Pro Asn Lys Val Gly Thr Cys
    130                 135                 140
Val Ser Glu Gly Ser Tyr Pro Ala Gly Thr Leu Ser Trp His Leu Asp
145                 150                 155                 160
Gly Lys Pro Leu Val Pro Asn Glu Lys Gly Val Ser Val Lys Glu Gln
                165                 170                 175
Thr Arg Arg His Pro Glu Thr Gly Leu Phe Thr Leu Gln Ser Glu Leu
            180                 185                 190
Met Val Thr Pro Ala Arg Gly Gly Asp Pro Arg Pro Thr Phe Ser Cys
        195                 200                 205
Ser Phe Ser Pro Gly Leu Pro Arg His Arg Ala Leu Arg Thr Ala Pro
    210                 215                 220

Ile Gln Pro Arg Val Trp Glu Pro Val Pro Leu Glu Glu Val Gln Leu
225                 230                 235                 240

Val Val Glu Pro Glu Gly Gly Ala Val Ala Pro Gly Gly Thr Val Thr
                245                 250                 255

Leu Thr Cys Glu Val Pro Ala Gln Pro Ser Pro Gln Ile His Trp Met
            260                 265                 270

Lys Asp Gly Val Pro Leu Pro Leu Pro Pro Ser Pro Val Leu Ile Leu
        275                 280                 285

Pro Glu Ile Gly Pro Gln Asp Gln Gly Thr Tyr Ser Cys Val Ala Thr
    290                 295                 300

His Ser Ser His Gly Pro Gln Glu Ser Arg Ala Val Ser Ile Ser Ile
305                 310                 315                 320

Ile Glu Pro Gly Glu Glu Gly Pro Thr Ala Gly Ser Val Gly Gly Ser
                325                 330                 335

Gly Leu Gly Thr Leu Ala Leu Ala Leu Gly Ile Leu Gly Gly Leu Gly
            340                 345                 350

Thr Ala Ala Leu Leu Ile Gly Val Ile Leu Trp Gln Arg Arg Gln Arg
        355                 360                 365

Arg Gly Glu Glu Arg Lys Ala Pro Glu Asn Gln Glu Glu Glu Glu Glu
    370                 375                 380

Arg Ala Glu Leu Asn Gln Ser Glu Glu Pro Glu Ala Gly Glu Ser Ser
385                 390                 395                 400

Thr Gly Gly Pro